# EUROPEAN PATENT SPECIFICATION

(11) **EP 2 512 526 B1**
(45) Date of publication and mention of the grant of the patent: **03.06.2020**
(21) Application number: 10795540.3
(22) Date of filing: 13.12.2010
(51) Int. Cl.: A61L 9/01, A61L 101/32, C11D 3/00, A61L 9/014, C11D 3/50

(54) **MALODOR CONTROL COMPOSITION HAVING A MIXTURE OF VOLATILE ALDEHYDES AND METHODS THEREOF**
GERUCHSBEKÄMPFUNGSZUSAMMENSETZUNG MIT EINER MISCHUNG AUS FLÜCHTIGEN ALDEHYDEN UND VERFAHREN DAFÜR
COMPOSITION DE SUPPRESSION DES MAUVAISES ODEURS POSSÉDANT UN MÉLANGE D'ALDÉHYDES VOLATILS ET PROCÉDÉS ASSOCIÉS

(30) Priority: 17.12.2009 US 287348 P
(43) Date of publication of application: 24.10.2012
(73) Proprietor: The Procter & Gamble Company, Cincinnati, OH 45202 (US)
(72) Inventor: WOO, Ricky, Ah-Man, Hamilton, Ohio 45011 (US); READNOUR, Christine, Marie, Fort Mitchell, Kentucky 41017 (US); OLCHOVY, Jason, John, West Chester, Ohio 45069 (US); MALANYAON, Michael-Vincent, Nario, Indian Springs, Ohio 45011 (US); LIU, Zaiyou, West Chester, Ohio 45069 (US); JACKSON, Rhonda, Jean, Cincinnati, Ohio 45215 (US)
(74) Representative: P&G Patent Belgium UK
(86) International application number: PCT/US2010/060043
(87) International publication number: WO 2011/084377

(56) References cited:
- WO-A1-03/033635
- WO-A1-2006/005007
- US-A- 6 103 678
- US-A1- 2010 111 889

## Description

### FIELD OF THE INVENTION

The present invention relates to a malodor control composition having a mixture of volatile aldehydes, and methods thereof. The malodor control composition is suitable for use in a variety of applications, including use in fabric and air freshening products.

### BACKGROUND OF THE INVENTION

Products for reducing or masking malodors are well known in the art and are widely described in patent literature. These products may be designed to work specifically in air or on fabrics or other surfaces. See, e.g., U.S. Patent Nos. 5,942,217; 5,955,093; and 6,033,679. However, not all odors are effectively controlled by products on the market as amine-based malodors such as fish and urine malodors, and sulfur-based malodors such as garlic, onion, foot, and fecal malodors are difficult to combat. Further, the time required for a composition to noticeably combat malodors may create consumer doubt as to a product's efficacy on malodors. For example, the consumer may leave the treated space before the product begins to noticeably reduce the malodor.

US2010/111889 is directed to a malodor system suitable for use in disposable articles, such as disposable cleaning wipes, baby wipes, or skin care wipes. The system may comprise an aldehyde, an ester, an ionone, and optionally a macrocyclic musk. The system may optionally include a perfume.

The difficulty in overcoming a broad range of malodors has spawned a diverse assortment of products to neutralize, mask, or contain the malodors. There remains a need for a fast acting malodor control composition that neutralizes malodors and is effective on a broad range of malodors, including amine-based and sulfur-based malodors, while not overpowering malodors with an overwhelming perfume.

### SUMMARY OF THE INVENTION

The invention provides a malodor control composition comprising an effective amount of a mixture of two or more volatile aldehydes for neutralizing a malodor, wherein said two or more volatile aldehydes are selected from the group consisting of 2-ethoxy benzylaldehyde, 2-isopropyl-5-methyl-2-hexenal, 5-methyl furfural, cinnamic aldehyde, floral super, florhydral, o-anisaldehyde, pino acetaldehyde, trans-4-decenal, and mixtures thereof.

There is further described a malodor control composition comprising an effective amount of a mixture of three or more volatile aldehydes for neutralizing a malodor, wherein said three or more volatile aldehydes have a VP of about 0.001 torr to about 0.100 torr, measured at 25°C.

The invention further provides a method of neutralizing an amine-base malodor comprising treating said malodor with a malodor control composition according to the invention; wherein at least 20% of said amine malodor is neutralized 40 seconds after treatment.

In yet another embodiment, there is provided a method of neutralizing a sulfur-based malodor comprising treating said malodor with a malodor control composition according to the invention; wherein at least 20% of said thiol malodor is neutralized 30 minutes after treatment.

### BRIEF DESCRIPTION OF THE DRAWINGS

Fig. 1 is a graph showing the performance of one embodiment of a malodor control composition, in accordance with the present invention, on a sulfur-based malodor.
Fig. 2 is a graph showing the performance of one embodiment of a malodor control composition, in accordance with the present invention, on an amine-based malodor.

### DETAILED DESCRIPTION OF THE INVENTION

The present invention relates to a malodor control composition having a mixture of volatile aldehydes for neutralizing malodors; and methods thereof.

"Malodor" refers to compounds generally offensive or unpleasant to most people, such as the complex odors associated with bowel movements.

"Neutralize" or "neutralization" refers to the ability of a compound or product to reduce or eliminate malodorous compounds. Odor neutralization may be partial, affecting only some of the malodorous compounds in a given context, or affecting only part of a malodorous compound. A malodorous compound may be neutralized by chemical reaction resulting in a new chemical entity, by sequestration, by chelation, by association, or by any other interaction rendering the malodorous compound less malodorous or non-malodorous. Odor neutralization may be distinguished from odor masking or odor blocking by a change in the malodorous compound, as opposed to a change in the ability to perceive the malodor without any corresponding change in the condition of the malodorous compound.

### I. Malodor Control Composition

The malodor control composition includes a mixture of volatile aldehydes and is designed to deliver genuine malodor neutralization and not function merely by covering up or masking odors. A genuine malodor neutralization provides a sensory and analytically measurable (e.g. gas chromatograph) malodor reduction. Thus, if the malodor control composition delivers a genuine malodor neutralization, the composition will reduce malodors in the vapor and/or liquid phase.

### 1. Volatile Aldehydes

The malodor control composition includes a mixture of volatile aldehydes that neutralize malodors in vapor and/or liquid phase via chemical reactions. Aldehydes that are partially volatile may be considered a volatile aldehyde as used herein. Volatile aldehydes may react with amine-based odors, following the path of Schiff-base formation. Volatiles aldehydes may also react with sulfur-based odors, forming thiol acetals, hemi thiolacetals, and thiol esters in vapor and/or liquid phase. It may be desirable for these vapor and/or liquid phase volatile aldehydes to have virtually no negative impact on the desired perfume character of a product.

Suitable volatile aldehydes may have a vapor pressure (VP) in the range of about 0.000133 mbar (0.0001 torr) to 133 mbar (100 torr), alternatively about 0.000133 mbar (0.0001 torr) to about 13.3 mbar (10 torr), alternatively about 0.00133 mbar (0.001 torr) to about 66.7 mbar (50 torr), alternatively about 0.00133 mbar (0.001 torr) to about 26.7 mbar (20 torr), alternatively about 0.00133 mbar (0.001 torr) to about 0.133 mbar (0.100 torr), alternatively about 0.00133 mbar (0.001 torr) to 0.08 mbar (0.06 torr), alternatively about 0.00133 mbar (0.001 torr) to 0.04 mbar (0.03 torr), alternatively about 0.00667 mbar (0.005 torr) to about 26.7 mbar (20 torr), alternatively about 0.0133 mbar (0.01 torr) to about 26.7 mbar (20 torr), alternatively about 0.0133 mbar (0.01 torr) to about 20 mbar (15 torr), alternatively about 0.0133 mbar (0.01 torr) to about 13.3 mbar (10 torr), alternatively about 0.0667 mbar (0.05 torr) to about 13.3 mbar (10 torr), measured at 25°C.

The volatile aldehydes may also have a certain boiling point (B.P.) and octanol/water partition coefficient (P). The boiling point referred to herein is measured under normal standard pressure of 101325 Pa (760 mmHg). The boiling points of many volatile aldehydes, at standard 101325 Pa (760 mm Hg) are given in, for example, "Perfume and Flavor Chemicals (Aroma Chemicals)," written and published by Steffen Arctander, 1969.

The octanol/water partition coefficient of a volatile aldehyde is the ratio between its equilibrium concentrations in octanol and in water. The partition coefficients of the volatile aldehydes used in the malodor control composition may be more conveniently given in the form of their logarithm to the base 10, logP. The logP values of many volatile aldehydes have been reported. See, e.g., the Pomona92 database, available from Daylight Chemical Information Systems, Inc. (Daylight CIS), Irvine, California. However, the logP values are most conveniently calculated by the "CLOGP" program, also available from Daylight CIS. This program also lists experimental logP values when they are available in the Pomona92 database. The "calculated logP" (ClogP) is determined by the fragment approach of Hansch and Leo (cf., A. Leo, in Comprehensive Medicinal Chemistry, Vol. 4, C. Hansch, P. G. Sammens, J. B. Taylor and C. A. Ramsden, Eds., p. 295, Pergamon Press, 1990). The fragment approach is based on the chemical structure of each volatile aldehyde, and takes into account the numbers and types of atoms, the atom connectivity, and chemical bonding. The ClogP values, which are the most reliable and widely used estimates for this physicochemical property, are preferably used instead of the experimental logP values in the selection of volatile aldehydes for the malodor control composition.

The ClogP values may be defined by four groups and the volatile aldehydes may be selected from one or more of these groups. The first group comprises volatile aldehydes that have a B.P. of about 250 °C or less and ClogP of about 3 or less. The second group comprises volatile aldehydes that have a B.P. of 250°C or less and ClogP of 3.0 or more. The third group comprises volatile aldehydes that have a B.P. of 250°C or more and ClogP of 3.0 or less. The fourth group comprises volatile aldehydes that have a B.P. of 250°C or more and ClogP of 3.0 or more. The malodor control composition may comprise any combination of volatile aldehydes from one or more of the ClogP groups.

In some embodiments, the malodor control composition of the present invention may comprises, by total weight of the malodor control composition, from about 0% to about 30% of volatile aldehydes from group 1, alternatively about 25%; and/or about 0% to about 10% of volatile aldehydes from group 2, alternatively about 10%; and/or from about 10% to about 30% of volatile aldehydes from group 3, alternatively about 30%; and/or from about 35% to about 60% of volatile aldehydes from group 4, alternatively about 35%.

Exemplary volatile aldehydes which may be used in a malodor control composition include, but are not limited to, Adoxal (2,6,10-Trimethyl-9-undecenal), Bourgeonal (4-t-butylbenzenepropionaldehyde), Lilestralis 33 (2-methyl-4-t-butylphenyl)propanal), Cinnamic aldehyde, cinnamaldehyde (phenyl propenal, 3-phenyl-2-propenal), Citral, Geranial, Neral (dimethyloctadienal, 3,7-dimethyl-2,6-octadien-1-al), Cyclal C (2,4-dimethyl-3-cyclohexen-1-carbaldehyde), Florhydral (3-(3-Isopropyl-phenyl)-butyraldehyde), Citronellal (3,7-dimethyl 6-octenal), Cymal, cyclamen aldehyde, Cyclosal, Lime aldehyde (Alpha-methyl-p-isopropyl phenyl propyl aldehyde), Methyl Nonyl Acetaldehyde, aldehyde C12 MNA (2-methyl-1-undecanal), Hydroxycitronellal, citronellal hydrate (7-hydroxy-3,7-dimethyl octan-1-al), Helional (alpha-methyl-3,4-(methylenedioxy)-hydrocinnamaldehyde, hydrocinnamaldehyde (3-phenylpropanal, 3-phenylpropionaldehyde), Intreleven aldehyde (undec-10-en-1-al), Ligustral, Trivertal (2,4-dimethyl-3-cyclohexene-1-carboxaldehyde), Jasmorange, satinaldehyde (2-methyl-3-tolylproionaldehyde, 4-dimethylbenzenepropanal), Lyral (4-(4-hydroxy-4-methyl pentyl)-3-cyclohexene-1-carboxaldehyde), Melonal (2,6-Dimethyl-5-Heptenal), Methoxy Melonal (6-methoxy-2,6-dimethylheptanal), methoxycinnamaldehyde (trans-4-methoxycinnamaldehyde), Myrac aldehyde isohexenyl cyclohexenyl-carboxaldehyde, trifernal ((3-methyl-4-phenyl propanal, 3-phenyl butanal), lilial, P.T. Bucinal, lysmeral, benzenepropanal (4-tert-butyl-alpha-methyl-hydrocinnamaldehyde), Dupical, tricyclodecylidenebutanal (4-Tricyclo5210-2,6decylidene-8butanal), Melafleur (1,2,3,4,5,6,7,8-octahydro-8,8-dimethyl-2-naphthaldehyde), Methyl Octyl Acetaldehyde, aldehyde C-11 MOA (2-mehtyl deca-1-al), Onicidal (2,6,10-trimethyl-5,9-undecadien-1-al), Citronellyl oxyacetaldehyde, Muguet aldehyde 50 (3,7-dimethyl-6-octenyl) oxyacetaldehyde), phenylacetaldehyde, Mefranal (3-methyl-5-phenyl pentanal), Triplal, Vertocitral dimethyl tetrahydrobenzene aldehyde (2,4-dimethyl-3-cyclohexene-1-carboxaldehyde), 2-phenylproprionaldehyde, Hydrotropaldehyde, Canthoxal, anisylpropanal 4-methoxy-alpha-methyl benzenepropanal (2-anisylidene propanal), Cylcemone A (1,2,3,4,5,6,7,8-octahydro-8,8-dimethyl-2-naphthaldehyde), and Precylcemone B (1-cyclohexene-1-carboxaldehyde).

Still other exemplary aldehydes include, but are not limited to, acetaldehyde (ethanal), pentanal, valeraldehyde, amylaldehyde, Scentenal (octahydro-5-methoxy-4,7-Methano-1H-indene-2-carboxaldehyde), propionaldehyde (propanal), Cyclocitral, beta-cyclocitral, (2,6,6-trimethyl-1-cyclohexene-1-acetaldehyde), Iso Cyclocitral (2,4,6-trimethyl-3-cyclohexene-1-carboxaldehyde), isobutyraldehyde, butyraldehyde, isovaleraldehyde (3-methyl butyraldehyde), methylbutyraldehyde (2-methyl butyraldehyde, 2-methyl butanal), Dihydrocitronellal (3,7-dimethyl octan-1-al), 2-Ethylbutyraldehyde, 3-Methyl-2-butenal, 2-Methylpentanal, 2-Methyl Valeraldehyde, Hexenal (2-hexenal, trans-2-hexenal), Heptanal, Octanal, Nonanal, Decanal, Lauric aldehyde, Tridecanal, 2-Dodecanal, Methylthiobutanal, Glutaraldehyde, Pentanedial, Glutaric aldehyde, Heptenal, cis or trans-Heptenal, Undecenal (2-, 10-), 2,4-octadienal, Nonenal (2-, 6-), Decenal (2-, 4-), 2,4-hexadienal, 2,4-Decadienal, 2,6-Nonadienal, Octenal, 2,6-dimethyl 5-heptenal, 2-isopropyl-5-methyl-2-hexenal, Trifernal, beta methyl Benzenepropanal, 2,6,6-Trimethyl-1-cyclohexene-1-acetaldehyde, phenyl Butenal (2-phenyl 2-butenal), 2.Methyl-3(p-isopropylphenyl)-propionaldehyde, 3-(p-isopropylphenyl)-propionaldehyde, p-Tolylacetaldehyde (4-methylphenylacetaldehyde), Anisaldehyde (p-methoxybenzene aldehyde), Benzaldehyde, Vernaldehyde (1-Methyl-4-(4-methylpentyl)-3-cyclohexenecarbaldehyde), Heliotropin (piperonal) 3,4-Methylene dioxy benzaldehyde, alpha-Amylcinnamic aldehyde, 2-pentyl-3-phenylpropenoic aldehyde, Vanillin (4-methoxy 3-hydroxy benzaldehyde), Ethyl vanillin (3-ethoxy 4-hydroxybenzaldehyde), Hexyl Cinnamic aldehyde, Jasmonal H (alpha-n-hexylcinnamaldehyde), Floralozone, (para-ethyl-alpha,alpha-dimethyl Hydrocinnamaldehyde), Acalea (p-methyl-alpha-pentylcinnamaldehyde), methylcinnamaldehyde, alpha-Methylcinnamaldehyde (2-methyl 3-pheny propenal), alpha-hexylcinnamaldehyde (2-hexyl 3-phenyl propenal), Salicylaldehyde (2-hydroxy benzaldehyde), 4-ethyl benzaldehyde, Cuminaldehyde (4-isopropyl benzaldehyde), Ethoxybenzaldehyde, 2,4-dimethylbenzaldehyde, Veratraldehyde (3,4-dimethoxybenzaldehyde), Syringaldehyde (3,5-dimethoxy 4-hydroxybenzaldehyde), Catechaldehyde (3,4-dihydroxybenzaldehyde), Safranal (2,6,6-trimethyl-1,3-diene methanal), Myrtenal (pin-2-ene-1-carbaldehyde), Perillaldehyde L-4(1-methylethenyl)-1-cyclohexene-1-carboxaldehyde), 2,4-Dimethyl-3-cyclohexene carboxaldehyde, 2-Methyl-2-pentenal, 2-methylpentenal, pyruvaldehyde, formyl Tricyclodecan, Mandarin aldehyde, Cyclemax, Pino acetaldehyde, Corps Iris, Maceal, and Corps 4322.

In one embodiment, the malodor control composition includes a mixture of two or more volatile aldehydes selected from the group consisting of 2-ethoxy Benzylaldehyde, 2-isopropyl-5-methyl-2-hexenal, 5-methyl Furfural, 5-methyl-thiophene-carboxaldehyde, Adoxal, p-anisaldehyde, Benzylaldehyde, Bourgenal, Cinnamic aldehyde, Cymal, Decyl aldehyde, Floral super, Florhydral, Helional, Lauric aldehyde, Ligustral, Lyral, Melonal, o-anisaldehyde, Pino acetaldehyde, P.T. Bucinal, Thiophene carboxaldehyde, trans-4-Decenal, trans trans 2,4-Nonadienal, Undecyl aldehyde, and mixtures thereof.

In some embodiments, the malodor control composition includes fast reacting volatile aldehydes. "Fast reacting volatile aldehydes" refers to volatile aldehydes that either (1) reduce amine odors by 20% or more in less than 40 seconds; or (2) reduce thiol odors by 20% or more in less than 30 minutes.

In one embodiment, the malodor control composition includes a mixture of the volatile aldehydes listed in Table 1 and referred to herein as Accord A.

**Table 1**

| Material | Wt. % | CAS Number | ClogP Group | VP(torr) @25°C | VP (mbar) @25°C |
|---|---|---|---|---|---|
| Intreleven Aldehyde | 5.000 | 112-45-8 | 3 | 0.060 | 0.080 |
| Florhydral | 10.000 | 125109-85-5 | 4 | 0.008 | 0.011 |
| Floral Super | 25.000 | 71077-31-1 | 3 | 0.030 | 0.040 |
| Scentenal | 10.000 | 86803-90-9 | 2 | 0.010 | 0.013 |
| Cymal | 25.000 | 103-95-7 | 4 | 0.007 | 0.009 |
| o-anisaldehyde | 25.000 | 135-02-4 | 1 | 0.032 | 0.043 |

In another embodiment, the malodor control composition includes a mixture of the volatile aldehydes listed in Table 2 and referred to herein as Accord B.

**Table 2**

| Material | Wt. % | CAS Number | ClogP Group | VP (torr) @25°C | VP (mbar) @25°C |
|---|---|---|---|---|---|
| Intreleven Aldehyde | 2.000 | 112-45-8 | 3 | 0.060 | 0.080 |
| Florhydral | 20.000 | 125109-85-5 | 4 | 0.008 | 0.011 |
| Floral Super | 10.000 | 71077-31-1 | 3 | 0.030 | 0.040 |
| Scentenal | 5.000 | 86803-90-9 | 2 | 0.010 | 0.013 |
| Cymal | 25.000 | 103-95-7 | 4 | 0.007 | 0.009 |
| Floralozone | 10.000 | 67634-14-4 | 4 | 0.005 | 0.007 |
| Adoxal | 1.000 | 141-13-9 | 4 | 0.007 | 0.009 |
| Methyl Nonyl Acetaldehvde | 1.000 | 110-41-8 | 3 | 0.030 | 0.040 |
| Melonal | 1.000 | 106-72-9 | 3 | 0.670 | 0.893 |
| o-anisaldehyde | 25.000 | 135-02-4 | 1 | 0.032 | 0.043 |

In another embodiment, the malodor control composition includes a mixture of about 71.2% volatile aldehydes, the remainder being other an ester and an alcohol perfume raw material. This mixture is listed in Table 3 and referred to herein as Accord C.

**Table 3**

| Material | Wt. % | CAS Number | ClogP Group | VP (torr) @25°C | VP (mbar) @25°C |
|---|---|---|---|---|---|
| Intreleven Aldehyde | 2.000 | 112-45-8 | 3 | 0.060 | 0.080 |
| Florhydral | 10.000 | 125109-85-5 | 4 | 0.008 | 0.011 |
| Floral Super | 5.000 | 71077-31-1 | 3 | 0.030 | 0.040 |
| Scentenal | 2.000 | 86803-90-9 | 2 | 0.010 | 0.013 |
| Cymal | 15.000 | 103-95-7 | 4 | 0.007 | 0.009 |
| Floralozone | 12.000 | 67634-14-4 | 4 | 0.005 | 0.007 |
| Adoxal | 1.000 | 141-13-9 | 4 | 0.007 | 0.009 |
| Methyl Nonyl Acetaldehyde | 1.000 | 110-41-8 | 3 | 0.030 | 0.040 |
| Melonal | 1.000 | 106-72-9 | 3 | 0.670 | 0.893 |
| Flor Acetate | 11.800 | 5413-60-5 | 1 | 0.060 | 0.080 |
| Frutene | 7.000 | 17511-60-3 | 4 | 0.020 | 0.027 |
| Helional | 5.000 | 1205-17-0 | 2 | 0.0005 | 0.0007 |
| Bourgeonal | 2.000 | 18127-01-0 | 4 | 0.004 | 0.005 |
| Linalool | 10.000 | 78-70-6 | 3 | 0.050 | 0.067 |
| Benzaldehyde | 0.200 | 100-52-7 | 1 | 1.110 | 1.480 |
| o-anisaldehyde | 15.000 | 135-02-4 | 1 | 0.320 | 0.430 |

Accords A, B, or C can be formulated in with other perfume raw materials in an amount, for example, of about 10% by weight of the malodor control composition. Additionally, the individual volatile aldehydes or a various combination of the volatile aldehydes can be formulated into a malodor control composition. In certain embodiments, the volatile aldehydes may be present in an amount up to 100%, by weight of the malodor control composition, alternatively from 1% to about 100%, alternatively from about 2% to about 100%, alternatively from about 3% to about 100%, alternatively about 50% to about 100%, alternatively about 70% to about 100%, alternatively about 80% to about 100%, alternatively from about 1% to about 20%, alternatively from about 1% to about 10%, alternatively from about 2% to about 20%, alternatively from about 3% to about 20%, alternatively from about 4% to about 20%, alternatively from about 5% to about 20%.

In some embodiments where volatility is not important for neutralizing a malodor, the present invention may include poly-aldehydes, for example, di-, tri-, tetra-aldehydes. Such embodiments may include laundry detergents, additive, and the like for leave-on, through the wash, and rinse-off type of applications.

### 2. Optional Ingredients

The malodor control composition may, optionally, include odor masking agents, odor blocking agents, and/or diluents. "Odor blocking" refers to the ability of a compound to dull the human sense of smell. "Odor-masking" refers to the ability of a compound to mask or hide a malodorous compound. Odor-masking may include a compound with a non-offensive or pleasant smell that is dosed such it limits the ability to sense a malodorous compound. Odor-masking may involve the selection of compounds which coordinate with an anticipated malodor to change the perception of the overall scent provided by the combination of odorous compounds. Exemplary diluents include dipropylene glycol methyl ether, and 3-methoxy-3-methyl-1-butanol, and mixtures thereof.

The malodor control composition may also, optionally, include perfume raw materials that solely provide a hedonic benefit (i.e. that do not neutralize malodors yet provide a pleasant fragrance). Suitable perfumes are disclosed in US 6,248,135.

For example, the malodor control composition may include a mixture of volatile aldehydes for neutralizing a malodor, perfume ionones, and a diluent. Alternatively, the malodor control composition may include 100% volatile aldehydes.

### II. Methods of Use

The malodor control composition of the present invention may be used for a wide variety of applications that neutralize malodors in the vapor and/or liquid phase. In some embodiments, the malodor control composition may be formulated for use in energized vapor phase systems. "Energized" as used herein refers to a system that operates by using an electrical energy source, such as a battery or electrical wall outlet, to emit a targeted active. For such systems, the VP of the volatile aldehyes may be about 0.00133 mbar (0.001 torr) to about 26.7 mbar (20 torr), alternatively about 0.0133 mbar (0.01 torr) to about 13.3 mbar (10 torr), measured at 25°C. One example of an energized vapor phase system is a liquid electric plug-in type air freshening device.

In some embodiments, the malodor control composition may be formulated for use in non-energized vapor phase systems. "Non-energized" as used herein refers to a system that emits a targeted active passively or without the need for an electrical energy source. Aerosol sprayers and traditional trigger/pump sprayers are considered non-energized systems. For such non-energized systems, the VP of the volatile aldehydes may be about 0.0133 mbar (0.01 torr) to about 26.7 mbar (20 torr), alternatively about 0.067 mbar (0.05 torr) to about 13.3 mbar (10 torr), measured at 25°C. Non-limiting examples of a non-energized vapor phase system are passive air freshening diffusers such as those known by the trade name Renuzit® Crystal Elements; and aerosol sprays such as fabric and air freshening sprays and body deodorants.

In other embodiments, the malodor control composition may be formulated for use in a liquid phase system. For such systems, the VP may be about 0 mbar (0 torr) to about 26.7 mbar (20 torr), alternatively about 0.000133 mbar (0.0001 torr) to about 13.3 mbar (10 torr), measured at 25°C. Non-limiting examples of a liquid phase system are liquid laundry products, such as laundry detergents and additives; dish detergents; personal hygiene products such as body washes, shampoos, conditioners.

The malodor control composition may also be formulated for use in substrates such as plastics, wovens, or non-wovens (e.g cellulose fibers for paper products). Such substrates may be used as pet food packaging; paper towels; tissues; trash bags; diapers; baby wipes; adult incontinence products; feminine hygiene products such as sanitary napkins and tampons. The malodor control composition may also be formulated for use in commercial or industrial systems such as in septic tanks or sewage treatment equipment.

### EXAMPLES

### Effect of volatile aldehydes on amine-based and sulfur-based malodors

Malodor standards are prepared by pipeting 1 mL of butylamine (amine-based malodor) and butanethiol (sulfur-based malodor) into a 1.2 liter gas sampling bag. The bag is then filled to volume with nitrogen and allowed to sit for at least 12 hours to equilibrate.

A 1 µL sample of each volatile aldehyde listed in Table 4 and each Accord (A, B, and C) listed in Tables 1 to 3 is pipeted into individual 10 mL silanized headspace vials. The vials are sealed and allowed to equilibrate for at least 12 hours. Repeat 4 times for each sample (2 for butylamine analysis and 2 for butanethiol analysis).

After the equilibration period, 1.5 mL of the target malodor standard is injected into each vial containing a volatile aldehyde or Accord sample. For thiol analysis, the samples are held at room temperature for 30 minutes prior to injection into the system. A 1 mL headspace syringe is used to inject 250 µL of each sample into the system for the thiol samples. For amine analysis, the samples are injected immediately into the system after the malodor is introduced. A 1 mL headspace syringe is used to inject 500 µL of each sample into the system for the amine samples. A GC pillow is used for the amine analysis to shorten the run times. Samples are then analyzed using a GC/MS with a DB-5, 20 m, 1 µm film thickness column with an MPS-2 autosampler equipment with static headspace function. Data is analyzed by ion extraction on each total ion current current (56 for thiol - 30 for amine) and the area is used to calculate the percent reduction from the malodor standard for each sample.

Table 4 shows the effect of certain volatile aldehydes on neutralizing amine-based and sulfur based malodors at 40 seconds and 30 minutes, respectively.

**Table 4**

| Perfume Raw Material (R-CHO) | At least 20% butylamine reduction at 40 seconds? | At least 20% butanethiol reduction at 30 minutes? |
|---|---|---|
| 2,4,5 Trimethoxy Benzaldehyde | No | No |
| 2,4,6-Trimethoxy-benzylaldehyde | No | No |
| 2-ethoxy benzylaldehyde | Yes | Yes |
| 2-isopropyl-5-methyl-2-hexenal | Yes | Yes |
| 2-methyl-3-(2-furyl)-propenal | No | No |
| 3,4,5 Trimethoxy Benzaldehyde | No | No |
| 3,4-Trimethoxy-benzylaldehyde | No | No |
| 4-tertbutyl benzylaldehyde | Yes | No |
| 5-methyl furfural | Yes | Yes |
| 5-methyl-thiophene-carboxaldehvde | No | Yes |
| Adoxal | Yes | No |
| Amyl cinnamic aldehyde | No | No |
| Benzylaldehyde | Yes | No |
| Bourgenal | No | Yes |
| Cinnamic aldehyde | Yes | Yes |
| Citronelyl Oxyacetaldehyde | No | No |
| Cymal | Yes | No |
| Decyl aldehyde | Yes | No |
| Floral Super | Yes | Yes |
| Florhydral | Yes | Yes |
| Floralozone | No | No |
| Helional | Yes | No |
| Hydroxycitronellal | No | No |
| Lauric aldehyde | Yes | No |
| Ligustral | Yes | No |
| Lyral | Yes | No |
| Melonal | Yes | No |
| Methyl nonyl acetaldehyde | No | No |
| o-anisaldehyde | Yes | Yes |
| p-anisaldehyde | Yes | No |
| Pino acetaldehyde | Yes | Yes |
| P.T. Bucinal | Yes | No |
| Thiophene Carboxaldehyde | Yes | No |
| Trans-4-decenal | Yes | Yes |
| Trans Trans 2,4-Nonadienal | Yes | No |
| Undecyl aldehyde | Yes | No |

Table 5 shows the percent reduction of butylamine and butaniethiol at 40 seconds and 30 minutes, respectively, for Accords A, B, and C.

**Table 5**

| Accord | % reduction of butylamine at 40 sec. | % reduction of butanethiol at 30 min. |
|---|---|---|
| Accord A | 76.58 | 25.22 |
| Accord B | 51.54 | 35.38 |
| Accord C | 65.34 | 24.98 |

### Sensory Test - effect of volatile aldehydes on a sulfur-based malodor

Place Presto™ skillet into fume hood and turn on to 121°C (250°F). Place 80 grams of Crisco® oil into skillet and cover with skillet lid. Allow 10 minutes for equilibration. Remove skillet lid and check oil temperature with thermometer. Place 50 grams of chopped, commercially prepared garlic in water into skillet. Cover skillet with lid. Cook for 2.5 minutes or until garlic is translucent, with a portion staring to turn brown but not burn. Remove garlic from the skillet. Place 5 grams of garlic in each of 4 Petri dishes. Place covers on each Petri dish.

Place each covered Petri dish into individual test chambers. Each test chamber is 39.25 inches wide, by 25 inches deep, by 21.5 inches high with a volume of 12.2 cubic feet (0.34 cubic meters). The test chamber can be purchased from Electro-Tech Systems, Glenside, PA. Each test chamber is equipped with a fan (Newark catalog #70K9932, 115 VAC, 90CFM) purchased from Newark Electronics, Chicago, IL.

Remove the lids of the Petri dishes to expose the malodor for a dwell time sufficient to provide an initial odor intensity grade of 70-80 (about 1 minute). Once the initial odor intensity grade has been reached in a test chamber, remove the Petri dish from the test chamber.

Next, 3 Noticeables® air freshening devices, marketed by P&G, are each filled with the Control composition shown in Table 6.

**Table 6**

| Material Name | Wt% |
|---|---|
| Benzaldehyde | 0.150 |
| Floralozone | 0.097 |
| Helional | 1.455 |
| Hydroxycitronellal | 3.880 |
| Ligustral Or Triplal | 1.028 |
| Esters | 12.950 |
| Ethers | 50.190 |
| Ketones | 3.010 |
| Lactones | 0.490 |
| Alcohols | 21.610 |
| Terpenes | 5.140 |

The devices are set to the low intensity position and plugged into 3 of the 4 test chambers. All doors on chamber are closed.

At 5, 15, 20, 30, 45, and 60 minutes, trained evaluators open each chamber, smell the chamber for malodor intensity, and assign a malodor score, based on the scale in Table 7. The chamber door is closed but not locked between sequential evaluators. The scores are tabulated and the average score for each time interval is recorded.

**Table 7**

| Expert Sensory Grader Malodor Evaluation Scale | |
|---|---|
| Score | Description corresponding to Score |
| 0 | No malodor present |
| 10 | Very slight malodor - "I think there is a malodor present" |
| 20 | Slight malodor - "I detect something but cannot identify specific malodor |
| 25 | Slight malodor |
| 50 | Moderate |
| 75 | Strong Malodor |
| 100 | Extremely Strong Malodor |

The above protocol is repeated using Prototype I shown in Table 8 (instead of Control composition) in the 3 Noticeables air freshening devices.

**Table 8**

| Material Name | Wt. % |
|---|---|
| Benzaldehyde | 0.135 |
| Floralozone | 0.087 |
| Helional | 1.310 |
| Hydroxycitronellal | 3.492 |
| Ligustral Or Triplal | 0.925 |
| o-anisaldehyde | 2.500 |
| Intreleven Aldehyde | 0.500 |
| Florhydral | 1.000 |
| Floral Super | 2.500 |
| Scentenal | 1.000 |
| Cymal | 2.500 |
| Esters | 11.662 |
| Ethers | 45.171 |
| Ketones | 2.705 |
| Lactones | 0.437 |
| Alcohols | 19.446 |
| Terpenes | 4.632 |

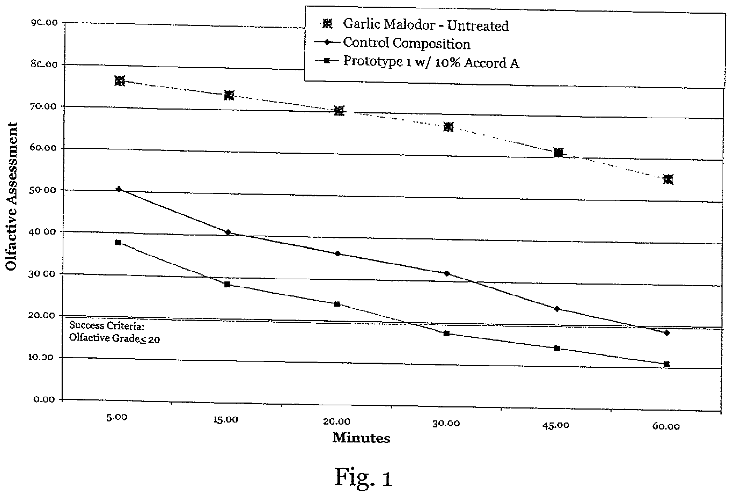

Fig. 1 shows that the formulation having 10% of the malodor control composition of the present invention reduces the garlic odor more than the Control composition that lacks such malodor control composition.

### Sensory test - effect of volatile aldehydes on an amine-based malodor

Separate fresh ocean perch fillets from skin and add to a Magic Bullet™ food chopper. Fish meat is chopped for 35-40 seconds. 25 grams of chopped fish is weighed and fashioned into a patty suitable to fit into a 60 x 15 mm Petri dish. Repeat 3 more times so there is one fish patty in each of 4 Petri dishes. Add 40g of Crisco® oil to Presto™ skillet. Place lid on skillet and turn on to 177°C (350°F). Allow 10 minutes for equilibration. Remove lid. Cut a slit in the middle of each patty, place 1 patty into skillet, and begin frying. Replace lid. After 2.5 minutes, flip fish patty and fry an additional 2.5 minutes. Remove fish patty from skillet and blot briefly onto a paper towel for 10 seconds. Fry the remaining 3 patties in the same manner. Place each fish patty into a 60 x 15mm Petri dish and cover with a lid.

Introduce each Petri dish containing a fish patty into individual test chambers. The specifications of the test chamber are the same as those in the above sulfur-based (i.e. garlic) malodor test. Remove the lids to expose the malodor for a dwell time sufficient for providing an initial odor intensity grade of 70-80 (about 1 minute). Once the initial odor intensity grade has been reached in a test chamber, remove the Petri dish from the test chamber.

Next, 3 Noticeables® air freshening devices, marketed by P&G, are each filled with the Control composition outlined in Table 6. The devices are set to the low intensity position and plugged into 3 of the 4 test chambers. All doors on chamber are closed.

At 5, 15, 20, 30, 45, and 60 minutes, trained evaluators open each chamber, smell the chamber for malodor intensity, and assign a malodor score, based on the scale in Table 7. The chamber door is closed but not locked between sequential evaluators. The scores are tabulated and the average score for each time interval is recorded.

The above protocol is repeated using Prototype I shown in Table 8 (instead of Control composition) in the 3 Noticeables® air freshening devices.

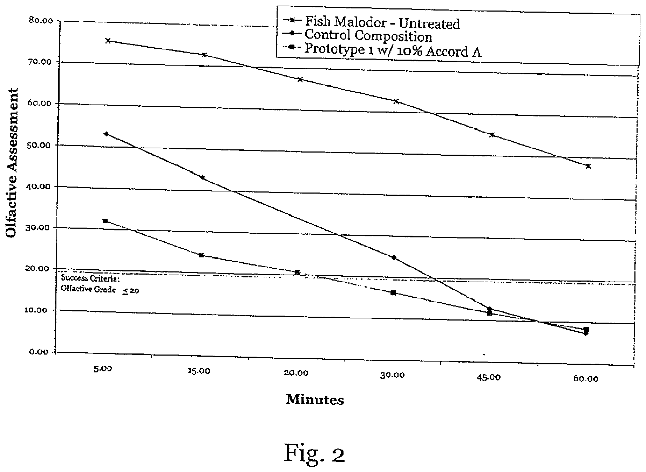

Fig. 2 shows that the formulation having 10% of the malodor control composition of the present invention reduces fish malodor more than the control composition that lacks such malodor control composition.

## Claims

1. A malodor control composition comprising an effective amount of a mixture of two or more volatile aldehydes for neutralizing a malodor, wherein said two or more volatile aldehydes are selected from the group consisting of 2-ethoxy benzylaldehyde, 2-isopropyl-5-methyl-2-hexenal, 5-methyl furfural, cinnamic aldehyde, floral super, florhydral, o-anisaldehyde, pino acetaldehyde, trans-4-decenal, and mixtures thereof.

2. A malodor control composition according to Claim 1, wherein said two or more volatile aldehydes comprise floral super and o-anisaldehyde.

3. The malodor control composition of Claim 1 or Claim 2 wherein said two or more volatile aldehydes have a vapor pressure (VP) from 0.00133 mbar (0.001 torr) to 0.133 mbar (0.100 torr).

4. The malodor control composition of Claim 1 or Claim 2 wherein said two or more volatile aldehydes comprise a mixture of volatile aldehydes selected from the group consisting of: Accord A, Accord B, Accord C, and mixtures thereof, and wherein Accord A, accord B and Accord C are defined as follows:
Accord A:
| Material | Wt. % | CAS Number |
|---|---|---|
| Intreleven Aldehyde | 5.000 | 112-45-8 |
| Florhydral | 10.000 | 125109-85-5 |
| Floral Super | 25.000 | 71077-31-1 |
| Scentenal | 10.000 | 86803-90-9 |
| Cymal | 25.000 | 103-95-7 |
| o-anisaldehyde | 25.000 | 135-02-4 |
Accord B:
| Material | Wt. % | CAS Number |
|---|---|---|
| Intreleven Aldehyde | 2.000 | 112-45-8 |
| Florhydral | 20.000 | 125109-85-5 |
| Floral Super | 10.000 | 71077-31-1 |
| Scentenal | 5.000 | 86803-90-9 |
| Cymal | 25.000 | 103-95-7 |
| Floralozone | 10.000 | 67634-14-4 |
| Adoxal | 1.000 | 141-13-9 |
| Methyl Nonyl Acetaldehyde | 1.000 | 110-41-8 |
| Melonal | 1.000 | 106-72-9 |
| o-anisaldehyde | 25.000 | 135-02-4 |
Accord C:
| Material | Wt. % | CAS Number |
|---|---|---|
| Intreleven Aldehyde | 2.000 | 112-45-8 |
| Florhydral | 10.000 | 125109-85-5 |
| Floral Super | 5.000 | 71077-31-1 |
| Scentenal | 2.000 | 86803-90-9 |
| Cvmal | 15.000 | 103-95-7 |
| Floralozone | 12.000 | 67634-14-4 |
| Adoxal | 1.000 | 141-13-9 |
| Methyl Nonyl Acetaldehyde | 1.000 | 110-41-8 |
| Melonal | 1.000 | 106-72-9 |
| Flor Acetate | 11.800 | 5413-60-5 |
| Frutene | 7.000 | 17511-60-3 |
| Helional | 5.000 | 1205-17-0 |
| Bourgeonal | 2.000 | 18127-01-0 |
| Linalool | 10.000 | 78-70-6 |
| Benzaldehyde | 0.200 | 100-52-7 |
| o-anisaldehyde | 15.000 | 135-02-4 |

5. The malodor control composition of Claim 1 or Claim 2 wherein said two or more volatile aldehydes comprise a mixture of volatile aldehydes comprising 1% to 10% of Accord A, by weight of said malodor control composition.

6. The malodor control composition of Claim 1 or Claim 2 wherein said composition has a pH of 4 to 6.5.

7. The malodor control composition of Claim 1 or Claim 2 further comprising an ingredient selected from the group consisting of: odor masking agents, odor blocking agents, diluents, and mixtures thereof.

8. A method of neutralizing an amine-base malodor comprising treating said malodor with the composition of Claim 1 or Claim 2 wherein at least 20% of said amine malodor is neutralized at 40 seconds after treatment, wherein the neutralization is determined as set out in the description under Effect of volatile aldehydes on amine-based and sulfur-based malodors.

9. The method of Claim 8 wherein said two or more volatile aldehydes have a VP of 0.00133 mbar (0.001 torr) to 0.040 mbar (0.03 torr).

10. A method of neutralizing a sulfur-based malodor comprising treating said malodor with the composition of Claim 1 or Claim 2 wherein at least 20% of said thiol malodor is neutralized 30 minutes after treatment, wherein the neutralization is determined as set out in the description under Effect of volatile aldehydes on amine-based and sulfur-based malodors.

11. The method of Claim 8 or Claim 10 wherein said composition comprises a mixture of volatile aldehydes selected from the group consisting of Accord A, Accord B, Accord C, and mixtures thereof, wherein Accord A, accord B and Accord C are defined as in Claim 4.

12. The method of claim of Claim 10 wherein said two or more volatile aldehydes have a VP of 0.013 to 20 mbar (0.01 to 15 torr).

## Patentansprüche

1. Geruchsbekämpfungszusammensetzung, umfassend eine wirksame Menge einer Mischung aus zwei oder mehreren flüchtigen Aldehyden zum Neutralisieren eines Geruchs, wobei die zwei oder mehreren flüchtigen Aldehyde ausgewählt sind aus der Gruppe bestehend aus 2-Ethoxybenzylaldehyd, 2-lsopropyl-5-methyl-2-hexenal, 5-Methylfurfural, Zimtaldehyd, Floral Super, Florhydral, o-Anisaldehyd, Pinoacetaldehyd, trans-4-Decenal und Mischungen davon.

2. Geruchsbekämpfungszusammensetzung nach Anspruch 1, wobei die zwei oder mehreren flüchtigen Aldehyde Floral Super und o-Anisaldehyd umfassen.

3. Geruchsbekämpfungszusammensetzung nach Anspruch 1 oder Anspruch 2, wobei die zwei oder mehreren flüchtigen Aldehyde einen Dampfdruck (VP) von 0,00133 mbar (0,001 Torr) bis 0,133 mbar (0,100 Torr) aufweisen.

4. Geruchsbekämpfungszusammensetzung nach Anspruch 1 oder Anspruch 2, wobei die zwei oder mehreren flüchtigen Aldehyde eine Mischung aus flüchtigen Aldehyden umfassen, ausgewählt aus der Gruppe bestehend aus: Accord A, Accord B, Accord C und Mischungen davon, und wobei Accord A, Accord B und Accord C wie folgt definiert sind:
Accord A:
| Material | Gew.-% | CAS-Nummer |
|---|---|---|
| Intreleven Aldehyd | 5,000 | 112-45-8 |
| Florhydral | 10,000 | 125109-85-5 |
| Floral Super | 25,000 | 71077-31-1 |
| Scentenal | 10,000 | 86803-90-9 |
| Cymal | 25,000 | 103-95-7 |
| o-Anisaldehyd | 25,000 | 135-02-4 |
Accord B:
| Material | Gew.-% | CAS-Nummer |
|---|---|---|
| Intreleven Aldehyd | 2,000 | 112-45-8 |
| Florhydral | 20,000 | 125109-85-5 |
| Floral Super | 10,000 | 71077-31-1 |
| Scentenal | 5,000 | 86803-90-9 |
| Cymal | 25,000 | 103-95-7 |
| Floralozon | 10,000 | 67634-14-4 |
| Adoxal | 1,000 | 141-13-9 |
| Methylnonylacetaldehyd | 1,000 | 110-41-8 |
| Melonal | 1,000 | 106-72-9 |
| o-Anisaldehyd | 25,000 | 135-02-4 |
Accord C:
| Material | Gew.-% | CAS-Nummer |
|---|---|---|
| Intreleven Aldehyd | 2,000 | 112-45-8 |
| Florhydral | 10,000 | 125109-85-5 |
| Floral Super | 5,000 | 71077-31-1 |
| Scentenal | 2,000 | 86803-90-9 |
| Cymal | 15,000 | 103-95-7 |
| Floralozon | 12,000 | 67634-14-4 |
| Adoxal | 1,000 | 141-13-9 |
| Methylnonylacetaldehyd | 1,000 | 110-41-8 |
| Melonal | 1,000 | 106-72-9 |
| Floracetat | 11,800 | 5413-60-5 |
| Fruten | 7,000 | 17511-60-3 |
| Helional | 5,000 | 1205-17-0 |
| Bourgeonal | 2,000 | 18127-01-0 |
| Linalool | 10,000 | 78-70-6 |
| Benzaldehyd | 0,200 | 100-52-7 |
| o-Anisaldehyd | 15,000 | 135-02-4 |

5. Geruchsbekämpfungszusammensetzung nach Anspruch 1 oder Anspruch 2, wobei die zwei oder mehreren flüchtigen Aldehyde eine Mischung aus flüchtigen Aldehyden umfassen, umfassend 1 Gew.-% bis 10 Gew.-% von Accord A der Geruchsbekämpfungszusammensetzung.

6. Geruchsbekämpfungszusammensetzung nach Anspruch 1 oder Anspruch 2, wobei die Zusammensetzung einen pH-Wert von 4 bis 6,5 aufweist.

7. Geruchsbekämpfungszusammensetzung nach Anspruch 1 oder Anspruch 2, ferner umfassend einen Bestandteil, ausgewählt aus der Gruppe bestehend aus: Geruchsmaskierungsmitteln, Geruchsblockiermitteln, Verdünnungsmitteln und Mischungen davon.

8. Verfahren zum Neutralisieren eines üblen Geruchs auf Aminbasis, umfassend Behandeln des üblen Geruchs mit der Zusammensetzung nach Anspruch 1 oder Anspruch 2, wobei mindestens 20 % des üblen Amin-Geruchs 40 Sekunden nach der Behandlung neutralisiert wird, wobei die Neutralisation bestimmt wird, wie in der Beschreibung unter Wirkung von flüchtigen Aldehyden auf üble Gerüche auf Aminbasis und Schwefelbasis dargelegt ist.

9. Verfahren nach Anspruch 8, wobei die zwei oder mehreren flüchtigen Aldehyde einen VP von 0,00133 mbar (0,001 Torr) bis 0,040 mbar (0,03 Torr) aufweisen.

10. Verfahren zum Neutralisieren eines üblen Geruchs auf Schwefelbasis, umfassend Behandeln des üblen Geruchs mit der Zusammensetzung nach Anspruch 1 oder Anspruch 2, wobei mindestens 20 % des üblen Thiol-Geruchs 30 Minuten nach der Behandlung neutralisiert wird, wobei die Neutralisation bestimmt wird, wie in der Beschreibung unter Wirkung von flüchtigen Aldehyden auf üble Gerüche auf Aminbasis und Schwefelbasis dargelegt ist.

11. Verfahren nach Anspruch 8 oder Anspruch 10, wobei die Zusammensetzung eine Mischung aus flüchtigen Aldehyden umfasst, ausgewählt aus der Gruppe bestehend aus Accord A, Accord B, Accord C und Mischungen davon, wobei Accord A, Accord B und Accord C wie in Anspruch 4 definiert sind.

12. Verfahren nach Anspruch nach Anspruch 10, wobei die zwei oder mehreren flüchtigen Aldehyde einen VP von 0,013 bis 20 mbar (0,01 bis 15 Torr) aufweisen.

## Revendications

1. Composition de lutte contre les mauvaises odeurs comprenant une quantité efficace d'un mélange de deux aldéhydes volatils ou plus pour neutraliser une mauvaise odeur, dans lequel lesdits deux aldéhydes volatils ou plus sont choisis dans le groupe constitué de 2-éthoxy benzylaldéhyde, 2-isopropyl-5-méthyl-2-hexénal, 5-méthyl-furfural, aldéhyde cinnamique, floral super, florhydral, o-anisaldéhyde, pino-acétaldéhyde, trans-4-décénal, et leurs mélanges.

2. Composition de lutte contre les mauvaises odeurs selon la revendication 1, dans laquelle lesdits deux aldéhydes volatils ou plus comprennent floral super et o-anisaldéhyde.

3. Composition de lutte contre les mauvaises odeurs selon la revendication 1 ou la revendication 2 dans laquelle lesdits deux aldéhydes volatils ou plus ont une pression de vapeur (PV) allant de 0,00133 mbar (0,001 torr) à 0,133 mbar (0,100 torr).

4. Composition de lutte contre les mauvaises odeurs selon la revendication 1 ou la revendication 2 dans laquelle lesdits deux aldéhydes volatils ou plus comprennent un mélange d'aldéhydes volatils choisis dans le groupe constitué de : Accord A, Accord B, Accord C, et leurs mélanges, et dans laquelle Accord A, accord B et Accord C sont définis comme suit :
Accord A :
| Matériau | % en poids | Numéro CAS |
|---|---|---|
| Intreleven aldéhyde | 5,000 | 112-45-8 |
| Florhydral | 10,000 | 125109-85-5 |
| Floral Super | 25,000 | 71077-31-1 |
| Scentenal | 10,000 | 86803-90-9 |
| Cymal | 25,000 | 103-95-7 |
| o-anisaldéhyde | 25,000 | 135-02-4 |
Accord B :
| Matériau | % en poids | Numéro CAS |
|---|---|---|
| Intreleven aldéhyde | 2,000 | 112-45-8 |
| Florhydral | 20,000 | 125109-85-5 |
| Floral Super | 10,000 | 71077-31-1 |
| Scentenal | 5,000 | 86803-90-9 |
| Cymal | 25,000 | 103-95-7 |
| Floralozone | 10,000 | 67634-14-4 |
| Adoxal | 1,000 | 141-13-9 |
| Méthyl-nonyl-acétaldéhyde | 1,000 | 110-41-8 |
| Melonal | 1,000 | 106-72-9 |
| o-anisaldéhyde | 25,000 | 135-02-4 |
Accord C :
| Matériau | % en poids | Numéro CAS |
|---|---|---|
| Intreleven aldéhyde | 2,000 | 112-45-8 |
| Florhydral | 10,000 | 125109-85-5 |
| Floral Super | 5,000 | 71077-31-1 |
| Scentenal | 2,000 | 86803-90-9 |
| Cymal | 15,000 | 103-95-7 |
| Floralozone | 12,000 | 67634-14-4 |
| Adoxal | 1,000 | 141-13-9 |
| Méthyl-nonyl-acétaldéhyde | 1,000 | 110-41-8 |
| Melonal | 1,000 | 106-72-9 |
| Flor Acetate | 11,800 | 5413-60-5 |
| Frutène | 7,000 | 17511-60-3 |
| Hélional | 5,000 | 1205-17-0 |
| Bourgeonal | 2,000 | 18127-01-0 |
| Linalol | 10,000 | 78-70-6 |
| Benzaldéhyde | 0,200 | 100-52-7 |
| o-anisaldéhyde | 15,000 | 135-02-4 |

5. Composition de lutte contre les mauvaises odeurs selon la revendication 1 ou la revendication 2 dans laquelle lesdits deux aldéhydes volatils ou plus comprennent un mélange d'aldéhydes volatils comprenant 1 % à 10 % d'Accord A, en poids de ladite composition de lutte contre les mauvaises odeurs.

6. Composition de lutte contre les mauvaises odeurs selon la revendication 1 ou la revendication 2 dans laquelle ladite composition a un pH de 4 à 6,5.

7. Composition de lutte contre les mauvaises odeurs selon la revendication 1 ou la revendication 2 comprenant en outre un ingrédient choisi dans le groupe constitué de : agents de masquage des odeurs, agents de blocage des odeurs, diluants, et leurs mélanges.

8. Procédé de neutralisation d'une mauvaise odeur à base d'amine comprenant le traitement de ladite mauvaise odeur avec la composition selon la revendication 1 ou la revendication 2 dans lequel au moins 20 % de ladite mauvaise odeur d'amine est neutralisée à 40 secondes après le traitement, dans lequel la neutralisation est déterminée de la façon décrite dans la description sous Effet d'aldéhydes volatils sur des mauvaises odeurs à base d'amine et à base de soufre.

9. Procédé selon la revendication 8 dans lequel lesdits deux aldéhydes volatils ou plus ont une PV de 0,00133 mbar (0,001 torr) à 0,040 mbar (0,03 torr).

10. Procédé de neutralisation d'une mauvaise odeur à base de soufre comprenant le traitement de ladite mauvaise odeur avec la composition selon la revendication 1 ou la revendication 2 dans lequel au moins 20 % de ladite mauvaise odeur de thiol est neutralisée à 30 minutes après le traitement, dans lequel la neutralisation est déterminée de la façon décrite dans la description sous Effet d'aldéhydes volatils sur des mauvaises odeurs à base d'amine et à base de soufre.

11. Procédé selon la revendication 8 ou la revendication 10 dans lequel ladite composition comprend un mélange d'aldéhydes volatils choisis dans le groupe constitué d'Accord A, Accord B, Accord C et leurs mélanges, dans lequel Accord A, accord B et Accord C sont tels que définis dans la revendication 4.

12. Procédé selon la revendication selon la revendication 10 dans lequel lesdits deux aldéhydes volatils ou plus ont une PV de 0,013 à 20 mbar (0,01 à 15 torr).
